# FASCICULE DE BREVET EUROPEEN

(11) **EP 2 205 630 B1**
(45) Date de publication et mention de la délivrance du brevet: **04.05.2016**
(21) Numéro de dépôt: 08839670.0
(22) Date de dépôt: 26.09.2008
(51) Int. Cl.: C07K 16/12, C12N 15/13, A61K 39/395, A61P 31/04, A61K 47/42, G01N 33/569

(54) **ANTICORPS CONTRE LES TOXINES DU CHARBON**
ANTIKÖRPER GEGEN ANTHRAXTOXINE
ANTIBODY AGAINST ANTHRAX TOXINS

(30) Priorité: 26.09.2007 FR 0706744
(43) Date de publication de la demande: 14.07.2010
(73) Titulaire: Etat Français représenté par le Délégué Général pour l' Armement, 00457 Armées (FR)
(72) Inventeur: THULLIER, Philippe, F-38190 Bernin (FR)
(74) Mandataire: Cabinet Plasseraud
(86) Numéro de dépôt international: PCT/FR2008/051726
(87) Numéro de publication internationale: WO 2009/050388

(56) Documents cités:
- WO-A-2007/084107
- US-A1- 2006 121 045
- LAFFLY EMMANUELLE ET AL: "Selection of a macaque Fab with framework regions like those in humans, high affinity, and ability to neutralize the protective antigen (PA) of Bacillus anthracis by binding to the segment of PA between residues 686 and 694" ANTIMICROBIAL AGENTS AND CHEMOTHERAPY, AMERICAN SOCIETY FOR MICROBIOLOGY, WASHINGTON, DC, US, vol. 49, no. 8, août 2005 (2005-08), pages 3414-3420, XP002444247 ISSN: 0066-4804 cité dans la demande
- WARK ET AL: "Latest technologies for the enhancement of antibody affinity" ADVANCED DRUG DELIVERY REVIEWS, AMSTERDAM, NL, vol. 58, no. 5-6, 7 août 2006 (2006-08-07), pages 657-670, XP005611420 ISSN: 0169-409X
- PRESTA ET AL: "Engineering of therapeutic antibodies to minimize immunogenicity and optimize function" ADVANCED DRUG DELIVERY REVIEWS, AMSTERDAM, NL, vol. 58, no. 5-6, 7 août 2006 (2006-08-07), pages 640-656, XP005611419 ISSN: 0169-409X
- LAFFLY E ET AL: "Improvement of an Antibody Neutralizing the Anthrax Toxin by Simultaneous Mutagenesis of Its Six Hypervariable Loops" JOURNAL OF MOLECULAR BIOLOGY, LONDON, GB, vol. 378, no. 5, 16 mai 2008 (2008-05-16), pages 1094-1103, XP022627585 ISSN: 0022-2836 [extrait le 2008-03-28]

## Description

La présente invention concerne des anticorps contre les toxines du charbon. En particulier, la présente invention a pour objet un anticorps anti PA, dirigé contre la sous-unité PA des toxines létale et oedémateuse du charbon, modifié de façon à présenter une affinité et une tolérance améliorées.

Le charbon est une maladie causée par un bacille Gram-positif, *Bacillus anthracis*. Cette bactérie est non mobile et forme des spores lorsqu'elle est placée dans un environnement défavorable à sa survie. Les spores peuvent survivre 24 heures dans l'air et environ 100 ans dans le sol, et possèdent des propriétés de résistance à la chaleur ou aux agents désinfectants.

Une infection par le charbon peut prendre trois formes : cutanée, pulmonaire ou digestive. L'infection pulmonaire est la plus fréquemment mortelle. En cas d'inhalation, les spores de *B. anthracis* passent dans les alvéoles où elles sont phagocytées par les macrophages et les cellules dendritiques, notamment. Les spores germent dans ces cellules et les formes végétatives se multiplient dans les ganglions. Les bactéries passent alors dans le sang, se reproduisent en continu et produisent des toxines, responsables en partie de la létalité de la maladie. Les toxines du charbon sont composées de trois protéines distinctes : l'antigène protecteur (PA, 83 kDa avant clivage enzymatique intracellulaire et 63 kDa après clivage), le facteur létal (LF, 90 kDa) et le facteur oedémateux (EF, 89 kDa). La toxine létale est formée de PA et LF ; et la toxine oedèmateuse, dont le rôle dans la physiologie de la maladie est moindre, de PA et EF. Ces protéines sont sécrétées par la bactérie en tant que monomères non toxiques, et s'assemblent à la surface des cellules cibles pour former des complexes toxiques. Jusqu'à présent, plusieurs antibiotiques, tels que la pénicilline, la doxycycline et les fluoroquinones, ont été utilisés pour le traitement des infections par le charbon. Cependant, certains de ces antibiotiques peuvent ne pas avoir d'effets sur certaines souches, résistantes aux antibiotiques. En particulier, certains de ces traitements pourraient ne pas être utilisables en cas de terrorisme ou guerre bactériologique, lorsque des souches résistantes aux antibiotiques pourraient être volontairement disséminées. De plus, comme les antibiotiques ne peuvent pas inhiber l'action de la toxine du charbon, il est nécessaire que ces antibiotiques soient administrés à des stades précoces de l'infection, or les diagnostics précoces sont difficiles à établir car les symptômes initiaux sont non spécifiques.

Des vaccins, dont le composant majeur est l'antigène protecteur PA, ont été développés mais ne sont utilisés que pour des personnes très fortement susceptibles d'être en contact avec *B. anthracis*. De plus, du fait de la nécessité d'une période de plusieurs mois pour acquérir une immunité suffisante, ces vaccins ne peuvent pas être utilisés dans des situations d'urgence. En France actuellement, aucun des ces vaccins n'est actuellement agréé pour l'utilisation humaine. Il est donc nécessaire de développer de nouvelles approches thérapeutiques et préventives, autres que les antibiotiques. L'immunisation passive avec des anticorps représente une stratégie efficace pour neutraliser la toxine. Plusieurs essais ont été réalisés pour neutraliser la toxine létale du charbon à l'aide d'anticorps monoclonaux contre l'antigène protecteur (PA) et le facteur létal (LF). La neutralisation de la toxine létale du charbon à l'aide d'un anticorps peut avoir lieu par inhibition de la liaison de PA et de son récepteur cellulaire, inhibition du clivage de PA, inhibition de la liaison entre PA et LF ou encore inhibition de l'action de LF par exemple.

La demande WO2007/084107 décrit des anticorps qui neutralisent la toxine PA.

Le développement de nouveaux anticorps permettant de neutraliser la toxine du charbon est ainsi d'un intérêt général pour une prévention et un traitement efficace du charbon. Dans un travail récent, les inventeurs ont immunisé un macaque avec l'antigène protecteur PA83 pour obtenir des anticorps destinés à traiter l'infection humaine par le charbon. A partir de la moelle osseuse, les inventeurs ont amplifié les gènes codant des fragments d'anticorps spécifiques de PA83 et les ont clonés pour obtenir une librairie. Un fragment de forte affinité (Kd=3,4 nM) et fortement neutralisant (concentration d'inhibition à 50% = 5,6 +/- 0,13 nM), désigné sous le nom de 35PA83, a ensuite été isolé (Laffly et al., antimicrobial agents and chemotherapy, 2005, 49(8) : 3414-3420). Les inventeurs ont démontré que le fragment d'immunoglobuline 35PA83 neutralise la toxine du charbon en empêchant l'interaction de PA avec son récepteur cellulaire.

Les inventeurs ont ensuite recherché à modifier ce fragment d'immunoglobuline pour en améliorer les qualités dans le but d'une utilisation médicale (prophylactique ou thérapeutique), en améliorant son affinité et sa tolérance par l'homme. Améliorer l'affinité de ce fragment d'immunoglobuline présente l'avantage d'utiliser une quantité plus faible d'immunoglobuline pour obtenir une activité biologique suffisante et permet aussi de réduire le coût du traitement. Améliorer la tolérance chez l'homme présente l'avantage d'éviter des réponses immunitaires dirigées contre ce fragment d'anticorps. La présente invention a donc pour objet de fournir un anticorps anti PA modifié à partir du fragment d'immunoglobuline 35PA83.

La présente invention a aussi pour objet une composition comprenant ledit anticorps modifié ainsi qu'une composition pharmaceutique comprenant ledit anticorps modifié. La présente invention a également pour objet l'utilisation dudit anticorps modifié pour la préparation d'une composition pharmaceutique destinée au traitement ou à la prévention d'une infection par le charbon.

La présente invention concerne également qu'une méthode de détection d'une toxine du charbon utilisant ledit anticorps modifié.

La présente invention sera mieux comprise à l'aide des définitions suivantes.

Le terme « anticorps » se réfère à une molécule d'immunoglobuline ou un fragment d'une molécule d'immunoglobuline ayant la capacité de se lier spécifiquement à un antigène particulier. Des fragments d'immunoglobuline bien connus sont par exemple les fragments F(ab')2, Fab, Fv, scFv et Fd.

Le terme « charbon » se réfère à toute maladie causée, directement ou indirectement, par une infection par *Bacillus anthracis*. Les symptômes initiaux d'une infection par inhalation ressemblent à ceux d'un rhume (fièvre, douleurs musculaires..). Après plusieurs jours, ces symptômes progressent vers des problèmes sévères de détresse respiratoire et de choc septique. L'inhalation du charbon est en générale fatale. L'infection cutanée par le charbon a lieu lorsque la bactérie entre dans la peau au niveau d'une brèche cutanée préexistante. Cette infection donne lieu dans un premier temps à une papule, qui se développe en deux-trois jours en une vésicule puis en un ulcère de 1-3 centimètres de diamètre présentant une aire nécrotique au centre. L'infection gastrointestinale par le charbon se développe suite à la consommation de viande contaminée et est caractérisée par une inflammation aigue du tractus intestinal.

Le terme « isolé » signifie « amplifié in vitro par PCR », « produit de façon recombinante par clonage », « purifié par séparation sur gel ou par clivage », ou encore « synthétisé par exemple par synthèse chimique ».

Le terme « vecteur » se réfère à un acide nucléique dans lequel la séquence d'intérêt peut être insérée par restriction puis ligation pour le transport entre différents environnements génétiques ou pour l'expression dans une cellule hôte. Les vecteurs sont par exemples les plasmides, les cosmides, les chromosomes artificiels de levures (YAC), les chromosomes artificiels de bactéries (BAC) et les chromosomes artificiels dérivés du bactériophage P1 (PAC), les vecteurs dérivés de virus. Un vecteur de clonage est un vecteur capable de se répliquer dans une cellule hôte et qui est de plus caractérisé par la présence de un ou plusieurs sites de restrictions par les endonucléases. Un vecteur d'expression est un vecteur dans lequel la séquence d'ADN d'intérêt peut être insérée par restriction ou ligation de telle façon qu'elle puisse être répliquée et/ou transcrite en ARN. Les vecteurs peuvent contenir en outre un ou plusieurs marqueurs de sélection ou d'identification des cellules ayant été transformées ou transfectées avec le vecteur.

Le terme « anticorps humanisé » se réfère à des anticorps d'origine animale dans lesquels des composants humains ont été substitués à certains composants originels.

Le terme «prévention d'une maladie » correspond à la prévention de l'apparition de cette maladie chez un sujet, en particulier un humain, chez qui la maladie ne s'est pas encore déclarée.

Le terme « traitement d'une maladie » correspond à l'inhibition de cette maladie, i.e. l'arrêt de son développement, sa régression, ou à la disparition des symptômes et conséquences de la maladie, ou encore à la disparition des causes de la maladie.

Le terme « quantité thérapeutique effective » se réfère à la quantité qui est suffisante pour effectuer le traitement lorsqu'elle est administrée à un sujet qui nécessite un tel traitement. La quantité thérapeutique effective dépend du sujet, du stade de la maladie à traiter et du mode d'administration, et peut être déterminée par des opérations de routine par l'homme du métier.

Comme il est bien connu, seule une partie de l'anticorps, la région variable, est impliquée dans la liaison de l'anticorps à son épitope. Les régions constantes de l'anticorps activent les effecteurs immunitaires, phagocytes ou cellules tueuses, et le complément ; ces régions constantes ne sont pas impliquées dans la liaison à l'antigène. Un anticorps dont la région constante (Fc) a été enzymatiquement clivée de façon à en préserver la région charnière est désigné comme un fragment F(ab')2 et conserve les deux sites de liaison à l'antigène.

De même, un anticorps dont la région constante, y compris la région charnière a été enzymatiquement clivée, ou qui a été produit sans cette région, est désigné comme un fragment Fab et conserve un des deux sites de liaison à l'antigène. Les fragments Fab consistent en une chaîne légère qui est liée de façon covalente à une portion de la chaîne lourde appelée Fd.

Dans la région variable, on trouve les régions déterminant la complémentarité (CDRs, *complementary determining regions*), aussi appelées régions hypervariables, qui interagissent directement avec l'antigène. Modifier les CDRs peut donc permettre de modifier l'affinité d'un anticorps. Dans la région variable, on trouve un second type de régions, appelées régions charpentes (FRs, *frameworks*), qui maintiennent la structure tertiaire des CDRs. Ces régions charpentes sont assez spécifiques de l'espèce où a été produit l'anticorps. Dans le fragment Fd de la chaîne lourde et dans la chaîne légère, on trouve quatre régions charpentes (FR1 à 4) séparées respectivement par trois CDR (CDR1 à 3).

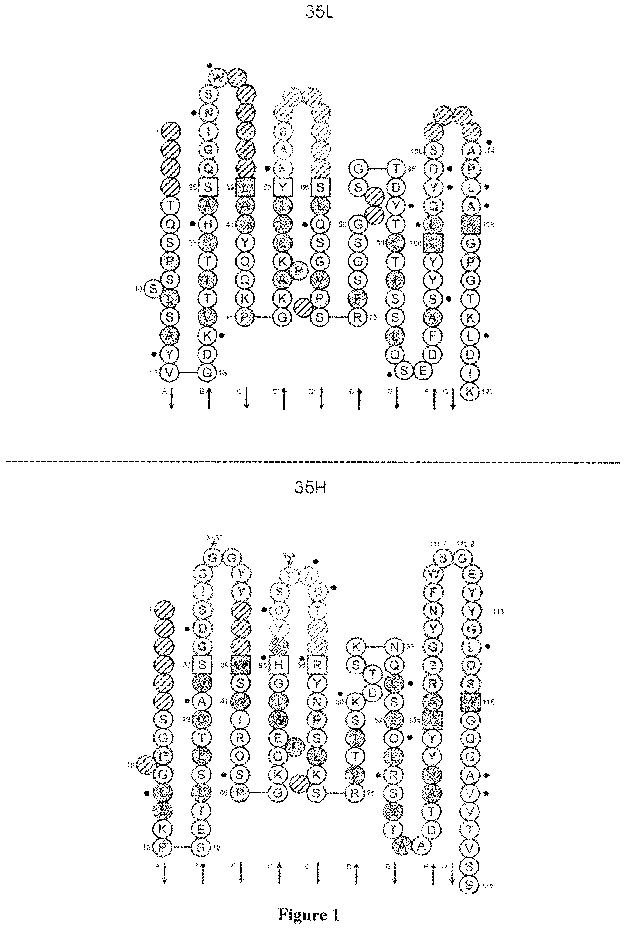

La présente invention a pour objet un anticorps anti PA comprenant :
- une partie Fc d'origine humaine, et :
   - ou bien la région variable de chaîne lourde de séquence d'acides aminés représentée par SEQ ID N°1 mutée aux positions suivantes : H/L (55) et S/G (74), et la région variable de chaîne légère de séquence d'acides aminés représentée par SEQ ID N°2 mutée en position suivante : Q/L (68),
   - ou bien la région variable de chaîne lourde de séquence d'acides aminés représentée par SEQ ID N°1 mutée aux positions suivantes : G/S (31A), R/K (66) et K/R (73), et la région variable de chaîne légère de séquence d'acides aminés représentée par SEQ ID N°2,
   les positions des acides aminés étant celles indiquées à la figure 1, et présentant une affinité supérieure à celle de l'anticorps non muté.

La séquence peptidique SEQ ID N°3, correspondant au fragment Fd de l'anticorps 35PA83, décrit dans Laffly et al., est accessible dans les banques informatisées, comme Genbank, sous le numéro CAH17920 et la séquence peptidique SEQ ID N°4, correspondant à la chaîne légère de l'anticorps 35PA83 est accessible de la même manière sous le numéro CAH17921.

Les régions variables de ces séquences sont présentées sous forme de schéma bidimensionnel sur la figure 1.

Les séquences d'ADN codant le fragment Fd et la chaîne légère de l'anticorps 35PA83, appelées ici SEQ ID N°5 et SEQ ID N°6, sont également accessibles dans les banques informatisées, sous les numéros AJ810486 et AJ810487, respectivement.

L'affinité K_{D} d'un anticorps peut être mesurée par les techniques conventionnelles connues de l'homme du métier.

L'anticorps non modifié (i.e. non muté) parental 35PA83 présente une affinité K_{D} égale à 3,4 10⁻⁹. Cette constante d'affinité a été calculé à partir des constantes d'association et de dissociation mesurées en temps réel par résonance plasmonique de surface tel qu'expliqué dans les exemples.

La mutation G/S (31A) dans la région variable de la chaîne lourde signifie que l'acide aminé G en position 31A (voir figure 1 pour les positions des acides aminés) a été substitué par l'acide aminé S.

Cet anticorps anti PA modifié par les mutations citées ci-dessus dans la région variable de la chaîne lourde et dans la région variable de la chaîne légère présente une affinité pour PA améliorée par rapport à celle de l'anticorps 35PA83.

Selon un mode de l'invention, la partie Fc de l'anticorps peut être choisie de façon à produire des IgA, des IgM ou des IgG.

Selon l'invention, l'anticorps anti PA modifié selon l'invention possède une partie Fc d'origine humaine. De tels anticorps entiers sont préférés pour l'administration chez l'homme car ils présentent une demi-vie plus longue que des fragments d'anticorps tels que les Fab, et sont plus adaptés à une administration intraveineuse, intra-péritonéale, intramusculaire, sous-cutanée ou transdermale.

D'après la description ci-dessus des séquences d'acides aminés de la région variable de la chaîne lourde et de la région variable de la chaîne légère des anticorps anti PA modifiées selon l'invention, l'homme du métier est capable de synthétiser, ou de faire synthétiser, des acides nucléiques qui codent ces séquences d'acides aminés.

La présente invention a donc pour objet un acide nucléique codant un anticorps anti PA modifié selon l'invention, qui présente une affinité améliorée par rapport à l'anticorps 35PA83 non muté.

La présente invention a également pour objet un vecteur comprenant ledit acide nucléique.

Ces acides nucléiques pourront être compris dans un vecteur recombinant pour le clonage ou pour l'expression des anticorps de l'invention.

La présente invention inclut tous les vecteurs recombinants contenant des séquences codantes pour la transformation, transfection ou thérapie génique eucaryote ou procaryote. De tels vecteurs pourront être préparés selon les techniques conventionnelles de biologie moléculaire et comprendront en outre un promoteur approprié, optionnellement une séquence signal pour l'export ou la sécrétion, et des séquences régulatrices nécessaires pour la transcription de la séquence nucléotidique. Un polypeptide de fusion peut être utile pour la purification des anticorps de la présente invention. Le domaine de fusion peut par exemple inclure une queue poly-histidine qui permet la purification sur des colonnes Ni+, ou une ancre membranaire de phage filamenteux qui est particulièrement utile pour le screening de banque, selon la technologie du « phage display ».

Un des vecteurs approprié dans le cadre de l'invention est une molécule d'ADN recombinante adaptée pour recevoir et exprimer une première et une seconde séquence d'ADN, de façon à permettre l'expression d'anticorps hétérodimérique tel qu'un anticorps de longueur complète ou des fragments F(ab')2 ou Fab selon l'invention. Un tel vecteur fournit un système pour indépendamment cloner les deux séquences d'ADN dans deux cassettes séparées présentes dans le vecteur, de façon à former deux cistrons séparés pour l'expression d'un premier et d'un second polypeptide de l'anticorps hétérodimérique. Un tel vecteur d'expression est appelé vecteur di-cistronique.

Les anticorps modifiés de la présente invention peuvent être produits dans des cellules eucaryotes telles que des CHO ou des hybridomes humain ou murin par exemple, ainsi que dans des cellules végétales.

La présente invention a également pour objet des cellules hôtes, procaryotes ou eucaryotes, comprenant un vecteur selon l'invention.

Un autre objet de la présente invention est de fournir une composition comprenant au moins un anticorps anti PA modifié selon l'invention pour en améliorer l' affinité.

La présente invention a également pour objet une composition pharmaceutique comprenant au moins un anticorps anti PA modifié selon l'invention pour en améliorer l'affinité.

Ladite composition pharmaceutique comprend de façon préférée un véhicule pharmaceutiquement acceptable. Un tel véhicule correspond au sens de l'invention à un matériel non toxique qui n'interfère pas avec l'efficacité de l'activité biologique des ingrédients actifs de la composition. Le terme « pharmaceutiquement acceptable » réfère à un matériel non toxique qui est compatible avec un système biologique tel qu'une cellule, une culture cellulaire, un tissu ou un organisme. Les caractéristiques du véhicule dépendront du mode d'administration.

La présente invention concerne l'utilisation d'au moins un anticorps anti-PA modifié pour en améliorer l'affinité selon l'invention pour la préparation d'une composition pharmaceutique ou d'un médicament destiné au traitement ou à la prévention d'une infection par *Bacillus anthracis*.

L'anticorps anti PA modifié pour en améliorer l'affinité selon l'invention peut être marqué. Des exemples de marqueurs comprennent les enzymes, les radio-isotopes, les composés fluorescents, les métaux colloïdes, les composés chimioluminescents, et les composés bioluminiscents. Les méthodes de liaison d'un marqueur à un anticorps sont bien connues de l'homme du métier.

Une autre technique de marquage consiste à coupler l'anticorps à des haptènes de faibles poids moléculaire, ces haptènes pouvant être spécifiquement modifiés au moyen d'une seconde réaction. Des exemples d'haptènes sont la biotine, qui réagit avec l'avidine, ou le dinitrophenol, le pyridoxal ou la fluorescéine, qui peuvent réagir avec des anticorps spécifiques anti-haptènes.

Est également décrit un kit pour la détection d'une toxine du charbon comprenant PA. Ce kit comprend :
- un container comprenant au moins un anticorps anti PA modifié selon l'invention pour en améliorer l'affinité et qui peut être ou non marqué,
- optionnellement, un container comprenant des solutions tampons
- et optionnellement un container comprenant des moyens de détection dudit anticorps anti PA modifié marqué, tel qu'une protéine de liaison à la biotine, par exemple l'avidine ou la streptavidine, liée à une molécule rapporteur, telle qu'un marqueur fluorescent ou enzymatique. Ce container peut aussi comprendre des moyens de détection dudit anticorps anti PA modifié non marqué, soit essentiellement des anticorps ou fragments d'anticorps.

L'anticorps anti PA modifié pour en améliorer l'affinité de l'invention peut être utilisé *in vitro,* par exemple dans des tests immunologiques dans lesquels ils sont utilisés en phase liquide ou liés à un véhicule de phase solide. Des exemples de véhicules bien connus sont le verre, le polystyrène, le polypropylène, le polyéthylène, le dextran, le nylon, l'amylase, la cellulose naturelle ou modifiée, le polyacrylamide, l'agarose ou la magnétite. Des exemples de tests immunologiques utilisant l'anticorps anti PA de l'invention sont des radioimmunoessais, des marquages histoimmunologiques, des ELISA, des western-blots, des essais d'immuno-précipitation, des essais d'immuno-diffusion, des essais de fixation du complément, des analyses au FACS ou encore des analyses par puces à protéines.

La présente invention a pour objet de fournir une méthode de détection *in vitro* d'une toxine du charbon, comprenant PA, dans un échantillon biologique, comprenant :
- la mise en contact de l'échantillon avec au moins un anticorps anti PA modifié selon l'invention pour en améliorer l'affinité et
- la détection de la liaison dudit anticorps comme indicateur de la présence de ladite toxine du charbon.

L'échantillon biologique peut être liquide : par exemple de la salive, de l'urine, du fluide cérébrospinal, du sérum ou du sang, ou solide ou semi-solide, par exemple des tissus ou des matières fécales ou un tissu solide tel qu'utilisé couramment en diagnostic histologique.

Est également décrite une méthode de détection *in vivo* d'une toxine du charbon comprenant PA, dans laquelle un anticorps anti PA modifié selon l'invention pour en améliorer l'affinité et marqué est administré à un sujet. La quantité d'anticorps modifié marqué administrée doit être suffisante pour permettre la détection de la liaison de l'anticorps à la toxine. La quantité d'anticorps modifié marqué administrée dépendra des facteurs tels que l'âge et le sexe du sujet, ainsi que du stade de la maladie. La quantité administrée peut varier entre 0.01 mg/kg et 50 mg/kg, préférablement entre 0.1 mg/kg et 20 mg/kg, et plus préférablement entre 0.1 mg/kg et 2 mg/kg.

Pour effectuer le diagnostic in vivo, l'anticorps anti PA modifié de l'invention doit être lié à un radioisotope directement ou indirectement par l'intermédiaire de groupes fonctionnels. Des groupes fonctionnels couramment utilisés sont par exemple l'acide diéthylène-triamine-pentacétique (DTPA) et l'acide éthylène-diamine-tétraacétique (EDTA). Des exemples d'ions métalliques radioisotopes sont ¹¹¹In, ⁹⁷Ru, ⁶⁷Ga, ⁶⁸Ga, ⁷²As, 89Zr et ²⁰¹T1.

Les anticorps anti PA modifiés de l'invention peuvent aussi être marqués avec un isotope paramagnétique pour le diagnostic par imagerie de résonance magnétique (IRM) ou par résonance de spin électronique (ESR). Des radioisotopes gamma émetteurs de positrons peuvent également être utilisés, tels que ¹⁵⁷Gd ⁵⁵Mn, ¹⁶²Dy, ⁶⁸Ga, ⁵²Cr, et ⁵⁶Fe.

Les anticorps anti PA modifiés pour en améliorer l'affinité de l'invention peuvent également être utilisés *in vitro* ou *in vivo* pour suivre l'évolution du traitement de la maladie, par exemple en déterminant l'augmentation ou la diminution du nombre de cellules ciblées par les toxines du charbon ou les changements dans la concentration de la toxine PA dans un échantillon biologique. Est également décrite une méthode de traitement d'un sujet, de préférence un humain, susceptible d'être infecté par *Bacillus anthracis*, dans laquelle une quantité thérapeutiquement efficace d'un anticorps anti PA modifié selon l'invention pour en améliorer l'affinité est administré audit sujet.

Une quantité thérapeutiquement efficace correspond à une quantité suffisante pour diminuer les symptômes de la maladie et l'évolution de l'infection. Cette quantité peut varier avec l'âge, le sexe du sujet et le stade de la maladie et sera déterminée par l'homme du métier. Une quantité thérapeutiquement efficace peut varier entre 0.01 mg/kg et 50 mg/kg, préférablement entre 0.1 mg/kg et 20 mg/kg, et plus préférablement entre 0.1 mg/kg et 2 mg/kg, en une ou plusieurs administrations quotidiennes, pendant un ou plusieurs jours.

Le mode d'administration peut être par injection ou par perfusion graduelle. L'injection peut être intraveineuse, intra-péritonéale, intramusculaire, sous-cutanée ou transdermale.

Les préparations pour une administration parentérale peuvent inclure des solutions aqueuses ou non-aqueuses stériles, des suspensions ou des émulsions. Des exemples de solvents non-aqueux sont le propylène glycol, le polyéthylène glycol, des huiles végétales, telle que l'huile d'olive, ou des esters organiques injectables tels que l'éthyl oléate. Des véhicules aqueux comprennent l'eau, des solutions alcool/eau, des émulsions ou des suspensions.

La présente invention a également pour objet un immunoconjugué comprenant un anticorps anti PA modifié selon l'invention pour en améliorer l'affinité et lié, de façon directe ou indirecte, à un agent thérapeutique.

De tels agents thérapeutiques comprennent des agents chimiques, des radionucléides, des agents immunothérapeutiques, des cytokines, des chimiokines, des toxines ou des inhibiteurs d'enzyme. Des exemples de toxines sont la chaîne A de la diphtérie, la chaîne A de l'exotoxine, la chaîne A de la ricin, la chaîne A de l'abrine, la chaîne A de la modeccin, l'alpha-sarcin, les protéines Aleurites fordii, les protéines dianthines, les protéines Phytolaca americana, l'inhibiteur momordica charantia, la curcine, la crotine, l'inhibiteur sapaonaria officinalis, la gélonine, la mitogélline, la restrictocine, la phénomycine, l'énomycine et le tricothecenes. Des exemples de radionucléide sont ²¹²Bi, ¹³¹I, ¹³¹In, ⁹⁰Y, et ¹⁸⁶Re.

La présente invention sera mieux comprise à l'aide du complément de description qui va suivre, qui se réfère à des exemples d'obtention d'anticorps anti PA.

Dans les exemples qui suivent, donnés à titre illustratif, il sera fait référence aux figures en annexe:
Figure 1 : schéma en collier de perles la région variable de la chaîne lourde et de la région variable de la chaîne légère de l'anticorps 35PA83.

La représentation IMGT en collier de perles est réalisée en accord avec la numérotation IMGT. Les points indiquent les différences entre les gènes humains les plus similaires à 35PA83, et 35PA83. Les cercles hachurés correspondent aux positions manquantes selon la numérotation IMGT.

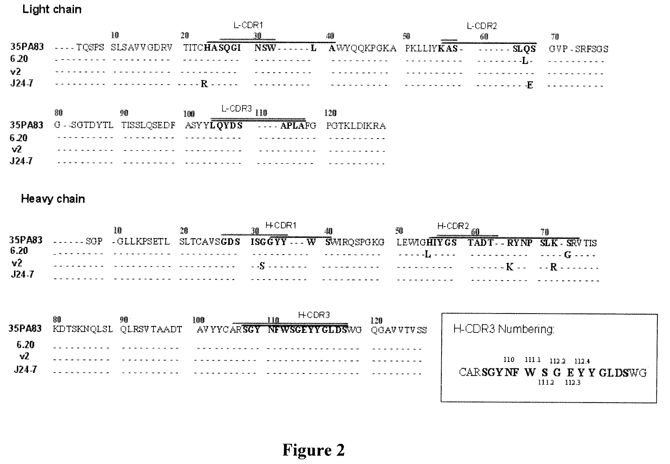

Figure 2 : alignement de séquence entre 3 anticorps anti PA modifiés selon l'invention (6.20, V2 et J24-7) et l'anticorps parental 35PA83

Le positionnement des CDRs suit la définition de l'IMGT (en gris clair) ou la définition de Kabat (en gris foncé, Wu and Kabat 1970). Les résidus sujets à mutation sont en gras. Tous les résidus sont numérotés selon la numérotation IMGT.

### Matériels et méthodes

### Souches E. coli

Les souches E. coli suivantes ont été utilisées :
- XL1 (Stratagène, La jolla, CA) : recA1 endA1 gyrA96 thi-1 hsdR17 sup E44 relA1 lac [F'proAB lacIqZΔM15 Tn10(Tetr)]
- SURE (Stratagène): e14(McrA) Δ(mcrCB-hsdSMR-mrr)171 endA1 supE44 thi-1 gyrA96 relA1 lac recB recJ sbcC umuC::Tn5 (Kanr) uvrC [F' proAB lacIqZΔM15 Tn10 (Tetr)]
- HB2151, utilisées pour l'expression de Fabs solubles.

### Toxins

Les toxines du charbon (PA83, LF et EF) ont été achetées chez List laboratories.

### Construction de la bibliothèque de mutants 35PA83

Une bibliothèque d'anticorps mutant dérivés du gène 35PA83 a été générée par Massive mutagenesis® (Biomethodes, Evry, France). Les mutations ont été introduites dans les CDRs des chaînes lourde et légère en utilisant des codons NNS. Les régions CDRs ont été définies selon Kabat et al. (Wu and Kabat 1970) et IMGT (Lefranc, Pommie et al. 2003).

La bibliothèque ADN a été utilisée pour transformer les cellules SURE par électroporation. Après ajout de milieu SB supplémenté en carbénicilline à la culture et incubation pendant 1h à 37°C, 1 ml de phage helper VCSM13 (environ 10¹² pfu) a été ajouté à la culture. Après incubation pendant 2h, 70 µg/ml de kanamycine ont été ajoutés et la culture a été mise sous agitation pendant la nuit à 37°C.

### Sélection d'anticorps par phage

Les particules de phages-Fab ont été purifiées et concentrées à partir de 50 ml de culture par précipitation au PEG, puis re-suspendues dans 3 ml de PBS-BSA 1%-azide 0,02% et filtrées sur un filtre de 0,45 µm. Le titre de cette préparation de phage était d'environ 5 10¹⁰ pfu/ml. Les phages-Fab ont été soumis à trois cycles d'infection-sélection-récupération tel que précédemment décrit (Andris-Widhopf, Rader et al. 2000). Certains phages ont été analysés, ou bien soumis à l'un ou bien à l'autre des processus décrits ci-dessous :

### Sélection par élution à très faible concentration d'antigène

La bibliothèque a été criblée en utilisant des concentrations décroissantes de PA83 biotinylé (100 nM, 10 nM, 1 nM, 0,1 nM, 0,01 nM, 0,001 nM) et des billes magnétiques recouvertes de streptavidine µMACS (Milteny Biotech). Différents mélanges de phages et d'antigène biotinylé, aux différentes concentrations, ont été incubés pendant 30 min à 37°C dans des tubes en verre. Les phages liés au PA83 biotinylé ont été capturés en utilisant 100 µl de billes magnétiques recouvertes de streptavidine µMACS (5 min à température ambiante). Après capture des phages, les billes ont été lavées 5 fois dans du PBS et une fois dans du TPBS (PBS contenant 0.1% de tween 20). Les phages liés ont ensuite été élués par incubation avec 100 µl de trypsine (10 µg/ml). L'éluat a été utilisé pour infecter les bactéries E. coli SURE en phase exponentielle de croissance.

### Sélection par incubation longue

Approximativement 10⁹-10¹⁰ phages par puits (50 µl) ont été criblés par liaison sur plaque (Nunc Maxisorp) recouverte avec 5 µg/ml de PA83 dans du PBS et bloquée avec 5% de MPBS. La plaque PA83 a été incubée avec les phages-Fab pendant 2h à 37°C et lavée 5 fois dans 0,1% Tween 20-PBS et 2 fois dans du PBS. Les phages ont ensuite été incubés à 4°C avec 50 µg/ml de PA83 soluble pendant des temps donnés (1 jour, 3, 13, 18, et 24 jours). Après 5 lavages, les phages liés ont été élués à 37°C pendant 15 min avec 50 µl de trypsine 10 µg/ml (Sigma) avant réinfection des bactéries E. coli SURE en phase exponentielle.

### Expression de Fab soluble, extraction périplasmique et purification

Chaque variant ADN a été transformé dans des bactéries de la souche d'E. *coli* appelée HB2151, rendues chimiquement compétentes. Les cellules ont été cultivées à 30°C, agitées à 250 rpm dans 1L de milieu SB contenant 50 µg/ml de carbénicilline et 0,1% de glucose. Lorsque la culture a atteint une A₆₀₀ de 1,5, une induction avec 1 mM d'IPTG a été effectuée pendant 18h à 22°C.

Les Fabs ont été extraits avec du sulfate de polymixine B (Sigma) et purifiés sur colonne de nickel (Ni-NTA spin column, QIAGEN, Valencia, CA) selon les instructions du fabricant, puis dialysés avec du PBS 1X à 4°C pendant 3h.

### Quantification du Fab soluble

La pureté du Fab a été testée par SDS-PAGE et sa concentration a été déterminée à l'aide du logiciel Quantity One® (Biorad).

### Mesure en temps réel de la résonance plasmonique de surface (SPR)

Les constantes de cinétique de l'interaction entre PA83 et les variants 35PA83 ont été déterminées en utilisant le système Biacore X SPR (BIAcore, Uppsala, Sweden). Le PA83 a été immobilisé sur une puce sensible CM5 en utilisant une procédure de couplage des aminés par injection de 30 µl de 2 µg/ml de PA83 dans 10 mM de sodium acétate pH 4,5. Pour minimiser la probabilité de reliaison, le K_{D} a été mesuré en utilisant un débit élevé (30 µl/min) et une quantité minimale d'antigène couplé (environ 500 RU, unités de résonance). Le taux de liaison de différentes concentrations de Fab allant de 5 à 400 nM dans du PBS a été déterminé à un débit de 30 µl/min. Les données de liaison ont été introduites dans un modèle langmuir 1 :1 du logiciel d'évaluation BIA. Les constantes d'association et de dissociation (kₒₙ et k_{off} respectivement) pour la liaison du Fab à PA83 ont été déterminées à 35°C.

### Analyse de séquences

Les séquences des chaînes lourde et légère des clones sélectionnés ont été déterminées par Genome Express (Meylan, France) en utilisant les amorces ompseq et newpelseq (Andris-Widhopf, Rader et al., 2000). Les séquences ont été analysées en ligne, en utilisant le système IMGT (http:/imgt.cines.fr).

### Test de neutralisation in vitro

La lignée cellulaire de macrophage murin J774A.1 a été incubée sur la nuit à une densité de 14 000 cellules par puits dans des plaques 96 puits. 400 ng/ml de PA et 40 ng/ml de LF ont été ajoutés simultanément au Fab ou au milieu seul et incubés pendant 1 heure à 37°C. Les mélanges ont ensuite été ajoutés aux macrophages et incubés pendant 4h à 37°C. Le Kit Promega CytoTox 96 Assay a ensuite été utilisé selon les instructions du fabricant pour déterminer les valeurs IC₅₀ du Fab pour la neutralisation LT (Pelat et al. 2007, Antimicrob. Agents Chemother 51, 2758-2764).

### Résultats

### Construction de la bibliothèque mutante de Fab (35PA83)

Une maturation d'affinité in vitro a été réalisée pour générer des nouveaux variants présentant une meilleure affinité. Dans ce but, une bibliothèque de variants a été générée par mutation exclusive des résidus de 6 CDRs, c'est-à-dire au niveau de 73 positions. La taille de la bibliothèque est de 5,4 10⁸ transformants. 45 clones plasmidiques indépendants ont été séquencés pour déterminer la diversité (tableau 1) et le taux de mutation. Le taux de mutation expérimental est de 3 mutations par fragment (V_{H}+V_{L}), ce qui permet une sélection directe des combinaisons de mutations qui augmentent l'affinité à PA.

L'examen de la fréquence mutationnelle à chaque position CDR ciblée dans la bibliothèque non sélectionnée comparé à la bibliothèque sélectionnée (tableau 1) a montré que certaines positions (en grisé) ne sont pas tolérantes à la variation. Ainsi, les résidus se trouvant à ces positions semblent être des résidus clés pour la liaison à l'antigène, préservant l'intégrité du site de liaison à l'antigène. En particulier, les résidus (H31-H40) du CDR1 sont définis comme des résidus de contact à l'antigène.

Il semble qu'une pression de sélection substantielle ait eu lieu pendant le processus de sélection car la bibliothèque non sélectionnée présente une plus grande diversité en comparaison avec les séquences sélectionnées, en particulier dans L-CDR1 et H-CDR1. Deux positions (en noir) sont fréquemment mutées dans la bibliothèque sélectionnée : H117 et L27. A l'exception du mutant J24-15, les mutations du résidu sérine H117 n'ont pas affecté les propriétés de liaison à l'antigène des Fabs (tableau 2). En revanche, les mutations des résidus Glutamine L27 semblent défavorables car elles diminuent l'affinité du Fab (mutant 6.7) ou affectent l'efficacité de l'expression du Fab (données non montrées).

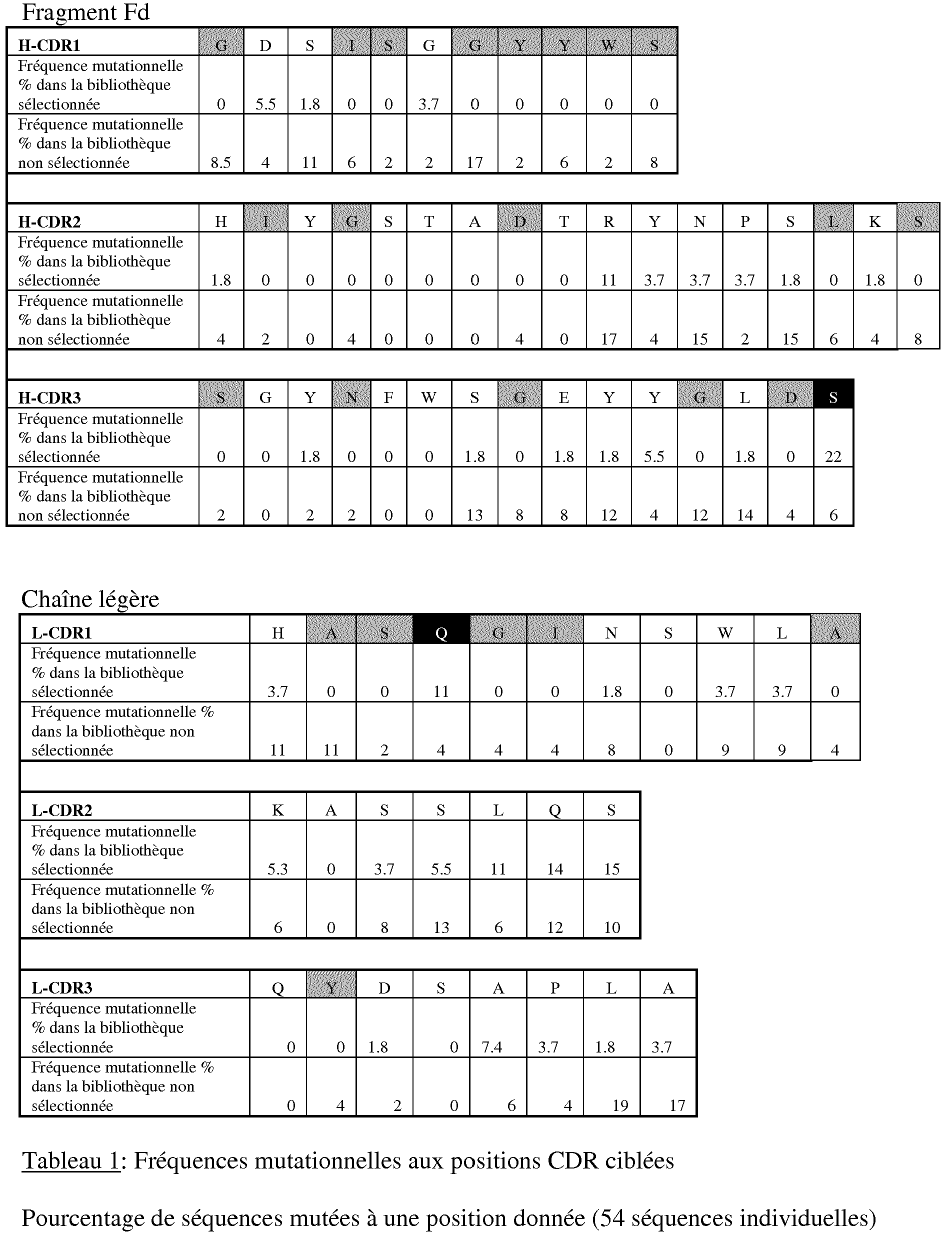

**Tableau 2: Affinité et cinétiques de liaison pour le Fab 35PA83 et ses variants.**

| **Clone parental** | **séquence H** | **séquence L** | **K_{D} (M)** | **kₒₙ(M⁻¹.s⁻¹)** | **k_{off}(s⁻¹)** |
|---|---|---|---|---|---|
| **35PA83** | parentale | parentale | 3,4 10⁻⁹ | 9,3 10⁴ | 3,2 10⁻⁴ |

| **Clones avec affinités nettement améliorées** | **séquence H** | **séquence** L | **K_{D} (M)** | **Kₒₙ(M⁻¹.s⁻¹)** | **k_{0ff}(s¹)** |
|---|---|---|---|---|---|
| **v2** | G>S (31A) | parentale | 6,6 10⁻¹⁰ | 1.22 10⁵ | 8,1 10⁻⁵ |
| | R>K (66) | | | | |
| | K>R(73) | | | | |
| 6.20 | H>L (55) | Q>L (68) | 1,8 10⁻¹⁰ | 2,86 10⁵ | 5.1 10⁻⁵ |
| | S>G (74) | | | | |
| **J24**-**7** | parentale | H>R (24) | 7,8 10⁻¹⁰ | 4,35 10⁵ | 3,4 10⁻⁴ |
| | | S>E (69) | | | |
| **J24**-**15** | S>L(117) | S>R (58) | 8,8 10⁻¹⁰ | 3,41 10⁵ | 3 10⁻⁴ |

| **Clones avec affinités dégradées** | **séquence H** | **séquence L** | **K_{D} (M)** | **kₒₙ(M⁻¹.s⁻¹)** | **k_{off}(s⁻¹)** |
|---|---|---|---|---|---|
| **v3** | parentale | L>R (67) | 2.27 10⁻⁸ | 8.98 10³ | 2 10⁻⁴ |
| **5.20** | parentale | S>R (69) | 1.22 10⁻⁸ | 2.4 10⁴ | 2.91 10⁴ |
| **6.7** | parentale | Q>V (27) | 8.11 10⁻⁹ | 1.9 10⁴ | 1.5 10⁻⁴ |
| | | S>L (66) | | | |
| **6.40** | parentale | H>R (24) | 1.10-8 | 1.9 10⁴ | 2.10⁻⁴ |

| **Clones avec affinités stables ou peu améliorées** | **séquence H** | **séquence L** | **K_{D} (M)** | **kₒₙ(M⁻¹.s⁻¹)** | **k_{off}(s⁻¹)** |
|---|---|---|---|---|---|
| **v8** | L>F (115) | A>S (117) | 2.54 10⁻⁹ | 9,2 10⁴ | 2.3 10⁻⁴ |
| **3.5** | parentale | Q>R (27) A>P (114) | 1,9 10⁻⁹ | 0,8 10⁵ | 1,5 10⁻⁴ |
| **5.7** | S>R (29) | W>D (32) | 2.57 10⁻⁹ | 5.5 10⁴ | 1_{.}4 10⁻⁴ |
| | S>R (117) | | | | |
| **5.15** | S>A (117) | parentale | 3.5 10⁻⁹ | 8.85 10⁴ | 3.11 10⁴ |
| **5.47** | Y>T (112.4) | P>S (115) | 2.11 10⁻⁹ | 8.81 10⁴ | 1.86 10⁻⁴ |
| **5.25** | parentale | Q>R (68) | 2 10⁻9 | 4,5 10⁴ | 9 10⁻⁵ |
| **5.39** | G>E (31A) D>G (28) | parentale | 1,71 10⁻⁹ | 0,63 10⁵ | 1,08 10⁻⁴ |
| **6.36** | Y>T (113) | P>S (115) | 2 10⁻⁹ | 0,62 10⁴ | 1.26 10⁻⁵ |
| **6.2** | S>G (74) | A>G (114) | 2 10⁻⁹ | 1,6 10⁵ | 3,2 10⁻⁴ |
| **6.46** | S>A (70) | S>R (69) | 1.82 10⁻⁹ | 6.9 10⁴ | 1.26 10⁴ |
| | E>Q (112.3) | | | | |
| | S>R (117) | | | | |
| **6.49** | P>G (69) | parentale | 1.55 10⁻⁹ | 9.6 10⁴ | 1.5 10⁻⁴ |
| | S>T (111.2) | | | | |
| **J24**-**3** | P>G (69) | parentale | 1,5 10⁻⁹ | 2 10⁵ | 2,9 10⁻⁴ |
| **J24**-**12** | parentale | A>D (114) | 3.5 10⁻⁹ | 7.8 10⁴ | 2.8 10⁻⁴ |
| **J24**-**13** | parentale | D>E (108) | 1.5 10⁻⁹ | 1.2 10⁵ | 1,86 10⁻⁴ |
| **J24**-**14** | D>G (28) | parentale | 3,3 10⁻⁹ | 6,14 10⁵ | 2,03 10⁻⁴ |
| | G>E (33) | | | | |
| | S>L(117) | | | | |

Les constantes d'association (kₒₙ) et de dissociation (k_{off}) ont été déterminées par résonance plasmonique de surface (BIAcore) and K_{D} a été calculé comme égal au rapport K_{off}/Kₒₙ.

### Criblage des variants

La bibliothèque mutante a été soumise à 3 cycles (R1, R2 et R3) d'infection-sélection-récupération. Après l'étape R3, 12 clones individuels ont été analysé par séquençage de V_{H} et V_{L}. Parmi ces 8 variants, 2 ont été trouvés identiques. 7 Fabs individuels ont donc été exprimés en tant que Fabs solubles. 3 d'entre eux ont été exprimés de façon suffisante pour permettre la mesure de l'affinité par SPR.

| | | | | |
|---|---|---|---|---|
| 12 séquences H+L | 4 parentaux | 34% | | |
| | 8 variants (2 identiques) | 66% | 3 clones exprimés | 37% |
| | | | 5 clones non exprimés | 63% |

Le triple mutant V2 a montré une constante de dissociation plus faible (k_{off}= 8,1 10⁵ s⁻¹) et une constante d'association un peu plus rapide (kₒₙ = 1,22 10⁵ M⁻¹.s⁻¹) que 35PA83, résultant en l'augmentation de l'affinité de 5,15 fois. Ce mutant présente 3 mutations dans le domaine variable de la chaîne lourde : une mutation (G31_{A}S) dans H-CDR1 et deux mutations dans H-CDR2 (R66K, K73R).

Après le troisième cycle, les phages ont été criblés selon deux processus supplémentaires de sélection : panning dans des puits recouverts de l'antigène avec incubation longue (« sélection par incubation longue ») ou bien en utilisant de l'antigène biotinylé soluble à très petite concentration (« sélection par élution à très faible concentration d'antigène soluble »).

**Sélection par élution à très faible concentration d'antigène soluble**

| Expérience | 1 | 2 | 3 | 4 | 5 | 6 | 7 | 8 | 9 |
|---|---|---|---|---|---|---|---|---|---|
| [PA83 biotinylé] nM | 100 | 10 | 1 | 0.1 | 0.01 | 0.001 | 0.0001 | 0.00001 | 0 |
| phages élués (x10³) | 250 | 192 | 55 | 10 | 8.8 | 3.4 | 1 | 0.7 | 0.9 |

La bibliothèque R3 a été sélectionnée en utilisant des concentrations décroissantes de PA83 biotinylé, allant de 100 nM à 0,01 pM. 1 pM représente la plus faible concentration permettant d'éluer davantage de clones que le contrôle négatif. Des clones individuels, obtenus à partir de la condition 6, ont été analysés dans un premier temps par séquences des chaînes lourdes et légères. Environ 35% d'entre eux présentaient des séquences sauvages.

### Condition 6

| | | | | |
|---|---|---|---|---|
| 43 séquences H+L | 15 parentaux | 35% | | |
| | 28 variants | 65% | Clones exprimés | 47% |
| | | | Clones non exprimés | 63% |

Parmi ces 28 variants, 47% ont été suffisamment exprimés pour permettre la mesure de l'affinité par SPR. Etant donné la redondance des clones, 8 variants individuels ont été exprimés et leurs constantes de cinétique ont été mesurées. Le fragment d'anticorps possédant la meilleure constante d'affinité (K_{D}=1,8 10⁻¹⁰ M, soit une amélioration d'un facteur égal à 18,9) est le triple mutant 6.20. Ce variant possède deux mutations dans le CDR2 de la chaîne lourde (H55L et S74G) et une mutation dans le CDR2 de la chaîne légère (Q68L).

**Sélection par incubation longue**

| Temps d'incubation (jour) | 3 | 13 | 18 | 24 | 25 |
|---|---|---|---|---|---|
| phages élués | 4000 | 5700 | 4000 | 18 | 0 |

| | | | | |
|---|---|---|---|---|
| 14 séquences H +L | 3 parentaux | 21% | | |
| | 11 variants | 78% | Clones exprimés | 72% |
| | | | Clones non exprimés | 27% |

Parmi les 18 clones élués au jour 24, 14 ont été entièrement séquencés (V_{H} et V_{L}) et seulement 3 étaient sauvages. 6 variants ont été suffisamment exprimés pour déterminer leurs constantes de cinétique. Les doubles mutants J24-7 (K_{D}=7,8 10⁻¹⁰ M) et J24-15 (K_{D}=8,8 10⁻¹⁰ M) ont montré une affinité de liaison augmentée d'un facteur 4,35 et 3,96 respectivement.

### Test de neutralisation LT in vitro

La concentration d'inhibition à 50% (IC₅₀) du Fab parent 35PA83 est de 5.6 nM.

La concentration d'inhibition à 50% a été mesurée pour les variants v2 et 6.20 : IC₅₀ du Fab v2 = 0.9 nM et IC₅₀ du Fab 6.20 = 3.3 nM.

Les variants v2et 6.20 présentent ainsi une meilleure capacité de neutralisation que le Fab parent 35PA83.

### Humanisation des variants

Les anticorps injectés à l'homme sont très bien tolérés lorsqu'ils sont humains. A l'inverse, les anticorps d'origine animale peuvent être cause d'effets secondaires délétères et sont éliminés rapidement. Les régions hypervariables des anticorps sont cependant tellement mutées pendant la maturation d'affinité qu'il est difficile, d'après la seule analyse de leurs séquences, d'en définir l'origine. Puisque les Fab ne comportent pas de région constante, les régions charpentes sont les seules régions impliquées dans leur tolérance.

Pour améliorer la tolérance du Fab 35PA83, et des molécules qui en seront dérivées, ce Fab a été humanisé. Une analyse automatique a été réalisée sur le serveur IMGT (http://imgt.cines.fr) et a permis de localiser, dans les régions charpentes du Fab 35PA83, les résidus différents de ceux codés par les gènes germinaux humains, codant les séquences les plus proches de celles de 35PA83. Des synthèses ou des mutations ponctuelles ont permis d'obtenir des séquences nucléotidiques codant des variants du Fab 35PA83 et comportant une, ou un petit nombre, de mutations augmentant l'homologie avec les séquences humaines. Les mutations qui ne causaient pas de dégradation significative de l'affinité, par rapport à 35PA83, figurent sur les tableaux 3 et 4. L'ensemble de ces mutations a été associé en un nouveau gène synthétique qui code pour un Fab dont les régions charpentes sont identiques à 97,75% à des régions charpentes codées par des gènes germinaux humains, contre 88,69% pour le Fab parental 35PA83. Toutefois, l'affinité de ce variant totalement humanisé (K_{D}=9 10⁻⁹ M) est dégradée d'un facteur 3 environ par rapport au Fab 35PA83.

**Tableau 3 : mutations des régions charpentes de la chaîne lourde**

| N° du résidu | 35PA83 | |
|---|---|---|
| 1 | aucun | Q |
| 2 | aucun | V |
| 3 | aucun | Q |
| 4 | aucun | L |
| 5 | aucun | Q |
| 6 | aucun | E |
| 12 | L | V |
| 24 | A | T |
| 45 | S | P |
| 66 | R | N |
| 80 | K | V |
| 87 | L | F |
| 92 | R | S |
| 122 | A | T |
| 123 | V | L |

**Tableau 4 : mutations des régions charpentes de la chaîne légère**

| N° du résidu | 35PA83 | |
|---|---|---|
| 1 | aucun | A |
| 2 | aucun | I |
| 3 | aucun | Q |
| 4 | aucun | L |
| 14 | Y | S |
| 18 | K | R |
| 24 | H | R |
| 87 | Y | F |
| 96 | S | P |
| 101 | S | T |
| 124 | L | V |

Les inventeurs ont recherché dans une nouvelle étude à déterminer quelles étaient les mutations dans les régions charpentes de la chaine lourde et de la chaine légère qui ne conduisaient pas à des modifications dans l'affinité du Fab.

Dans un premier temps, les mutations décrites dans les tableaux 5 et 6 suivants ont été réalisées dans les régions charpentes 1 et 4 de la chaine lourde et légère de 35PA83.

Le variant obtenu par ces mutations est appelé Hu₁35PA83.

**Tableau 5 : mutations des régions charpentes 1 et 4 de la chaîne lourde de 35PA83.**

| N° du résidu | 35PA83 | |
|---|---|---|
| 1 | aucun | Q |
| 2 | aucun | V |
| 3 | aucun | Q |
| 4 | aucun | L |
| 5 | aucun | Q |
| 6 | aucun | E |
| 12 | L | V |
| 24 | A | T |
| 122 | A | T |
| 123 | V | L |

**Tableau 5 : mutations des régions charpentes 1 et 4 de la chaîne légère de 35PA83**

| N° du résidu | 35PA83 | |
|---|---|---|
| 1 | aucun | A |
| 2 | aucun | I |
| 3 | aucun | Q |
| 4 | aucun | L |
| 14 | Y | S |
| 18 | K | R |
| 24 | H | R |
| 124 | L | V |

Les constantes de dissociation Kd ont été mesurées par Bioacore pour 35PA83 et le variant Hu₁35PA83. Les résultats obtenus sont très proches : 3,40 nM pour 35PA83 et 2,86 nM pour Hu₁PA83.

Ainsi, les mutations réalisées dans les régions charpentes 1 et 4 de la chaine lourde et de la chaine légère de 35PA83 n'affectent pas l'affinité de 35PA83 pour son antigène.

Dans un second temps, des mutations ont été réalisées dans les régions charpentes 2 et 3 de la chaine lourde et de la chaine légère de 35PA83. Ces mutations sont présentées dans les tableaux 6 et 7 suivants.

Le variant obtenu par les mutations décrites précédemment dans les régions charpentes 1 et 4 et par les mutations décrites ci-dessous dans les régions charpentes 2 et 3 a été appelé Hu₄PA83.

**Tableau 6 : mutations des régions charpentes 2 et 3de la chaîne lourde de 35PA83**

| N° du résidu | 35PA83 | |
|---|---|---|
| 45 | S | P |
| 66 | R | N |
| 87 | L | F |
| 92 | R | S |

**Tableau 7 : mutations des régions charpentes 2 et 3 de la chaîne légère de 35PA83**

| N° du résidu | 35PA83 | |
|---|---|---|
| 87 | Y | F |
| 96 | S | P |
| 101 | S | T |

Les constantes de dissociation Kd ont été mesurées par Bioacore pour 35PA83 et le variant Hu₄35PA83. Les résultats obtenus sont très proches : 3,40 nM pour 35PA83 et 3,72 nM pour Hu₁PA83.

Ainsi, les mutations réalisées dans les régions charpentes 1, 2, 3 et 4 de la chaine lourde et de la chaine légère de 35PA83 telles que décrites dans les tableaux 4 à 7 n'affectent pas l'affinité de 35PA83 pour son antigène.

Un test de neutralisation LT a également été réalisé selon le protocole décrit précédemment. La concentration inhibitrice 50% mesurée pour Hu₄35PA83 est de 5.8 nM tandis que celle mesurée pour 35PA83 est de 5.6 nM.

Ainsi, les mutations réalisées dans les régions charpentes 1, 2, 3 et 4 de la chaine lourde et de la chaine légère de 35PA83 telles que décrites dans les tableaux 4 à 7 n'affectent pas la capacité de neutralisation de 35PA83.

## Revendications

1. Anticorps anti antigène protecteur de la toxine du charbon comprenant :
- une partie Fc d'origine humaine, et :
- ou bien la région variable de chaîne lourde de séquence d'acides aminés représentée par SEQ ID N°1 mutée aux positions suivantes : H/L (55) et S/G (74), et la région variable de chaîne légère de séquence d'acides aminés représentée par SEQ ID N°2 mutée en position suivante : Q/L (68),
- ou bien la région variable de chaîne lourde de séquence d'acides aminés représentée par SEQ ID N°1 mutée aux positions suivantes : G/S (31A), R/K (66) et K/R (73), et la région variable de chaîne légère de séquence d'acides aminés représentée par SEQ ID N°2,
les positions des acides aminés étant celles indiquées à la figure 1, et **caractérisé en ce que** ledit anticorps modifié présente une affinité supérieure à celle de l'anticorps non muté.

2. Acide nucléique codant un anticorps anti antigène protecteur de la toxine du charbon selon la revendication **1.**

3. Vecteur comprenant un acide nucléique selon la revendication **2**.

4. Cellule hôte comprenant un vecteur selon la revendication **3.**

5. Composition comprenant au moins un anticorps anti antigène protecteur de la toxine du charbon selon la revendication **1.**

6. Composition pharmaceutique comprenant au moins un anticorps anti antigène protecteur de la toxine du charbon selon la revendication **1.**

7. Méthode pour la détection *in vitro* d'une toxine du charbon comprenant l'antigène protecteur de la toxine du charbon dans un échantillon biologique, comprenant :
- la mise en contact de l'échantillon avec au moins un anticorps anti antigène protecteur de la toxine du charbon selon la revendication **1,** et
- la détection de la liaison dudit anticorps comme indicateur de la présence de ladite toxine du charbon.

8. Immunoconjugué comprenant un anticorps anti antigène protecteur de la toxine du charbon selon la revendication **1** lié à un agent thérapeutique.

## Patentansprüche

1. Antikörper gegen das Schutzantigen von Anthraxtoxin, umfassend:
- einen Teil Fc mit humanem Ursprung und
- oder auch die variable Region der schweren Kette der Aminosäuresequenzen, die durch die SEQ ID NO:1 dargestellt wird, welche in folgenden Positionen mutiert ist: H/L (55) und S/G (74), und den variablen Bereich der leichten Kette der Aminosäuresequenz, dargestellt durch SEQ ID NO: 2, mutiert in der folgenden Position: Q/L (68),
oder auch die variable Region der schweren Kette der Aminosäuresequenzen, die durch die SEQ ID NO:1 dargestellt wird, welche in folgenden Positionen mutiert ist: G/S (31 A), R/K (66) und K/R (73), und den variablen Bereich der leichten Kette der Aminosäuresequenz, dargestellt durch SEQ ID NO:2,
wobei die Positionen der Aminosäuren die in Figur 1 dargestellten sind und **dadurch gekennzeichnet sind, dass** der modifizierte Antikörper gegenüber dem nichtmutierten Antikörper eine überragende Affinität zeigt.

2. Nukleinsäure, die für einen Antikörper nach Anspruch 1 codiert, der gegen das Schutzantigen von Anthraxtoxin gerichtet ist.

3. Vektor, umfassend eine Nukleinsäure nach Anspruch 2.

4. Wirtszelle, umfassend einen Vektor nach Anspruch 3.

5. Zusammensetzung, umfassend mindestens einen Antikörper gegen das Schutzantigen von Anthraxtoxin nach Anspruch 1.

6. Pharmazeutische Zusammensetzung, umfassend mindestens einen Antikörper gegen das Schutzantigen von Anthraxtoxin nach Anspruch 1.

7. Verfahren zum *in vitro*-Nachweis eines Anthraxtoxins, umfassend das Schutzantigen von Anthraxtoxin in einer biologischen Probe, umfassend:
- In-Kontakt-bringen der Probe mit mindestens einem Antikörper gegen das Schutzantigen von Anthraxtoxin nach Anspruch 1 und
- Nachweis der Bindung des Antikörpers als Indikator des Vorliegens des Anthraxtoxins.

8. Immunokonjugat, umfassend einen Antikörper gegen das Schutzantigen des Anthraxtoxins nach Anspruch 1, verbunden mit einem therapeutischen Mittel.

## Claims

1. An antibody anti-protective antigen of anthrax toxin comprising:
- a Fc part of human origin, and:
- either the variable region of the heavy chain of amino acid sequence represented by SEQ ID No.1 mutated on the following positions: H/L (55) and S/G (74), and the variable region of the light chain of amino acid sequence represented by SEQ ID No.2 mutated on the following position: Q/L (68),
- or the variable region of the heavy chain of amino acid sequence represented by SEQ ID No.1 mutated on the following positions: G/S (31A), R/K (66) and K/R (73), and the variable region of the light chain of amino acid sequence represented by SEQ ID No.2,
the positions of amino acids being indicated on figure 1, and **characterized in that** said modified antibody has improved affinity relative to the nonmutated antibody.

2. Nucleic acid coding for an antibody anti-protective antigen of anthrax toxin as claimed in claim 1.

3. Vector comprising a nucleic acid as claimed in claim 2.

4. Host cell comprising a vector as claimed in claim 3.

5. Composition comprising at least one antibody anti-protective antigen of anthrax toxin as claimed in claim 1.

6. Pharmaceutical composition comprising at least one antibody anti-protective antigen of anthrax toxin as claimed in claim 1.

7. A method for the *in vitro* detection of an anthrax toxin comprising the protective antigen of anthrax toxin in a biological sample, comprising:
- contacting the sample with at least one antibody anti-protective antigen of anthrax toxin as claimed in claim 1, and
- detecting the binding of said antibody as an indicator of the presence of said anthrax toxin.

8. Immunoconjugate comprising an antibody anti-protective antigen of anthrax toxin as claimed in claim 1 bound to a therapeutic agent.


### SEQUENCE LISTING

<110> Etat français, représenté par le Délégué Général de l'Armement
   THULLIER, Philippe
<120> ANTICORPS CONTRE LES TOXINES DU CHARBON
<130> BEX080300
<160> 22
<170> PatentIn version 3.3
<210> 1
   <211> 119
   <212> PRT
   <213> Macaca fascicularis
<400> 1
   Ser Gly Pro Gly Leu Leu Lys Pro Ser Glu Thr Leu Ser Leu Thr Cys
   1               5                   10                  15
   Ala Val Ser Gly Asp Ser Ile Ser Gly Gly Tyr Tyr Trp Ser Trp Ile
               20                  25                  30
   Arg Gln Ser Pro Gly Lys Gly Leu Glu Trp Ile Gly His Ile Tyr Gly
           35                  40                  45
   Ser Thr Ala Asp Thr Arg Tyr Asn Pro Ser Leu Lys Ser Arg Val Thr
       50                  55                  60
   Ile Ser Lys Asp Thr Ser Lys Asn Gln Leu Ser Leu Gln Leu Arg Ser
   65                  70                  75                  80
   Val Thr Ala Ala Asp Thr Ala Val Tyr Tyr Cys Ala Arg Ser Gly Tyr
                   85                  90                  95
   Asn Phe Trp Ser Gly Glu Tyr Tyr Gly Leu Asp Ser Trp Gly Gln Gly
               100                 105                 110
   Ala Val Val Thr Val Ser Ser
           115
<210> 2
   <211> 103
   <212> PRT
   <213> Macaca fascicularis
<400> 2
   Thr Gln Ser Pro Ser Ser Leu Ser Ala Tyr Val Gly Asp Lys Val Thr
   1               5                   10                  15
   Ile Thr Cys His Ala Ser Gln Gly Ile Asn Ser Trp Leu Ala Trp Tyr
               20                  25                  30
   Gln Gln Lys Pro Gly Lys Ala Pro Lys Leu Leu Ile Tyr Lys Ala Ser
           35                  40                  45
   Ser Leu Gln Ser Gly Val Pro Ser Arg Phe Ser Gly Ser Gly Ser Gly
       50                  55                  60
   Thr Asp Tyr Thr Leu Thr Ile Ser Ser Leu Gln Ser Glu Asp Phe Ala
   65                  70                  75                  80
   Ser Tyr Tyr Cys Leu Gln Tyr Asp Ser Ala Pro Leu Ala Phe Gly Pro
                   85                  90                  95
   Gly Thr Lys Leu Asp Ile Lys
               100
<210> 3
   <211> 223
   <212> PRT
   <213> Macaca fascicularis
<400> 3
   Gly Pro Gly Leu Leu Lys Pro Ser Glu Thr Leu Ser Leu Thr Cys Ala
   1               5                   10                  15
   Val Ser Gly Asp Ser Ile Ser Gly Gly Tyr Tyr Trp Ser Trp Ile Arg
               20                  25                  30
   Gln Ser Pro Gly Lys Gly Leu Glu Trp Ile Gly His Ile Tyr Gly Ser
           35                  40                  45
   Thr Ala Asp Thr Arg Tyr Asn Pro Ser Leu Lys Ser Arg Val Thr Ile
       50                  55                  60
   Ser Lys Asp Thr Ser Lys Asn Gln Leu Ser Leu Gln Leu Arg Ser Val
   65                  70                  75                  80
   Thr Ala Ala Asp Thr Ala Val Tyr Tyr Cys Ala Arg Ser Gly Tyr Asn
                   85                  90                  95
   Phe Trp Ser Gly Glu Tyr Tyr Gly Leu Asp Ser Trp Gly Gln Gly Ala
               100                 105                 110
   Val Val Thr Val Ser Ser Ala Ser Thr Lys Gly Pro Ser Val Phe Pro
           115                 120                 125
   Leu Ala Pro Ser Ser Arg Ser Thr Ser Glu Ser Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ser Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Val Cys Asn Val Asn His Lys Pro Ser
           195                 200                 205
   Asn Thr Lys Val Asp Lys Arg Val Glu Ile Lys Thr Cys Gly Gly
       210                 215                 220
<210> 4
   <211> 209
   <212> PRT
   <213> Macaca fascicularis
<400> 4
   Thr Gln Ser Pro Ser Ser Leu Ser Ala Tyr Val Gly Asp Lys Val Thr
   1               5                   10                  15
   Ile Thr Cys His Ala Ser Gln Gly Ile Asn Ser Trp Leu Ala Trp Tyr
               20                  25                  30
   Gln Gln Lys Pro Gly Lys Ala Pro Lys Leu Leu Ile Tyr Lys Ala Ser
           35                  40                  45
   Ser Leu Gln Ser Gly Val Pro Ser Arg Phe Ser Gly Ser Gly Ser Gly
       50                  55                  60
   Thr Asp Tyr Thr Leu Thr Ile Ser Ser Leu Gln Ser Glu Asp Phe Ala
   65                  70                  75                  80
   Ser Tyr Tyr Cys Leu Gln Tyr Asp Ser Ala Pro Leu Ala Phe Gly Pro
                   85                  90                  95
   Gly Thr Lys Leu Asp Ile Lys Arg Ala Val Ala Pro Pro Ser Val Phe
               100                 105                 110
   Ile Phe Pro Pro Ser Glu Asp Gln Val Thr Ser Gly Thr Val Ser Val
           115                 120                 125
   Val Cys Leu Leu Asn Asn Phe Tyr Pro Arg Glu Ala Ser Val Lys Trp
       130                 135                 140
   Lys Val Asp Gly Ala Leu Lys Thr Gly Asn Ser Gln Glu Ser Val Thr
   145                 150                 155                 160
   Glu Gln Asp Ser Lys Asp Asn Thr Tyr Ser Leu Ser Ser Thr Leu Thr
                   165                 170                 175
   Leu Ser Ser Thr Glu Tyr Gln Ser His Lys Val Tyr Ala Cys Glu Val
               180                 185                 190
   Thr His Gln Gly Leu Ser Ser Pro Val Thr Lys Ser Phe Asn Arg Gly
           195                 200                 205
   Glu
<210> 5
   <211> 670
   <212> DNA
   <213> Macaca fascicularis
<400> 5
   cgggcccagg actgctgaag ccttcggaaa ccctgtccct cacctgcgct gtctctggtg     60
   actccatcag cggtggttac tactggagct ggatccgcca gtccccaggg aaggggctgg    120
   agtggattgg gcatatctat ggtagtactg cggacaccag gtacaacccc tccctcaaga    180
   gtcgagtcac catttcaaaa gacacgtcca agaaccagct ctccctgcaa ctgaggtctg    240
   tgaccgccgc ggacacggcc gtgtattatt gtgcgagatc gggttacaat ttttggagtg    300
   gtgaatatta cggtttggat tcctggggcc aaggggccgt cgtcaccgtc tcctcagcct    360
   ccaccaaggg cccatcggtc ttccccctgg cgccctcctc caggagcacc tccgagagca    420
   cagcggccct gggctgcctg gtcaaggact acttccccga acccgtgacc gtgtcgtgga    480
   actcaggctc cctgaccagc ggcgtgcaca ccttcccggc tgtcctacag tcctcagggc    540
   tctactccct cagcagcgtg gtgaccgtgc cctccagcag cttgggcacc cagacctacg    600
   tctgcaacgt aaaccacaag cccagcaaca ccaaggtgga caagagagtt gagatcaaaa    660
   catgtggtgg                                                           670
<210> 6
   <211> 634
   <212> DNA
   <213> Macaca fascicularis
<400> 6
   acccagtctc catcgtccct gtctgcatat gtgggagaca aagtcaccat cacttgccat     60
   gccagtcagg gtattaacag ttggttagcc tggtatcagc agaaaccagg gaaagcccct    120
   aaacttctga tctataaggc gtccagtttg caaagtgggg tcccatcaag gttcagcggc    180
   agtggatctg ggacagatta tactctcacc atcagcagct tgcagtctga agactttgct    240
   tcttattact gtctacaata tgacagtgcc ccattggctt tcggccccgg gaccaagctg    300
   gatatcaaac gggctgtggc tccaccatct gtcttcatct tcccgccatc tgaagatcag    360
   gtgacatctg gaactgtctc tgttgtgtgc ctgctgaata acttctatcc cagagaggcc    420
   agcgtaaagt ggaaggtgga tggtgccctc aaaacgggta actcccagga gagtgtcaca    480
   gagcaggaca gcaaggacaa cacctacagc ctgagcagca ccctgacgct gagcagcaca    540
   gagtaccaga gtcacaaagt ctatgcctgc gaagtcaccc atcagggcct gagttcgccc    600
   gtcacaaaga gcttcaacag gggagagtgt taat                               634
<210> 7
   <211> 125
   <212> PRT
   <213> artificial
<220>
   <223> VH V2 variant
<400> 7
   Gln Val Gln Leu Gln Glu Ser Gly Pro Gly Leu Leu Lys Pro Ser Glu
   1               5                   10                  15
   Thr Leu Ser Leu Thr Cys Ala Val Ser Gly Asp Ser Ile Ser Ser Gly
               20                  25                  30
   Tyr Tyr Trp Ser Trp Ile Arg Gln Ser Pro Gly Lys Gly Leu Glu Trp
           35                  40                  45
   Ile Gly His Ile Tyr Gly Ser Thr Ala Asp Thr Lys Tyr Asn Pro Ser
       50                  55                  60
   Leu Arg Ser Arg Val Thr Ile Ser Lys Asp Thr Ser Lys Asn Gln Leu
   65                  70                  75                  80
   Ser Leu Gln Leu Arg Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Ser Trp Gly Gln Gly Ala Val Val Thr Val Ser Ser
           115                 120                 125
<210> 8
   <211> 107
   <212> PRT
   <213> artificial
<220>
   <223> VL V2 variant
<400> 8
   Ala Ile Gln Leu Thr Gln Ser Pro Ser Ser Leu Ser Ala Tyr Val Gly
   1               5                   10                  15
   Asp Lys Val Thr Ile Thr Cys His Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Gln Ser Gly Val Pro Ser Arg Phe Ser Gly
       50                  55                  60
   Ser Gly Ser Gly Thr Asp Tyr Thr Leu Thr Ile Ser Ser Leu Gln Ser
   65                  70                  75                  80
   Glu Asp Phe Ala Ser Tyr Tyr Cys Leu Gln Tyr Asp Ser Ala Pro Leu
                   85                  90                  95
   Ala Phe Gly Pro Gly Thr Lys Leu Asp Ile Lys
               100                 105
<210> 9
   <211> 125
   <212> PRT
   <213> artificial
<220>
   <223> VH 6.20 variant
<400> 9
   Gln Val Gln Leu Gln Glu Ser Gly Pro Gly Leu Leu Lys Pro Ser Glu
   1               5                   10                  15
   Thr Leu Ser Leu Thr Cys Ala Val Ser Gly Asp Ser Ile Ser Gly Gly
   20 25 30
   Tyr Tyr Trp Ser Trp Ile Arg Gln Ser Pro Gly Lys Gly Leu Glu Trp
   35 40 45
   Ile Gly Leu Ile Tyr Gly Ser Thr Ala Asp Thr Arg Tyr Asn Pro Ser
   50 55 60
   Leu Lys Gly Arg Val Thr Ile Ser Lys Asp Thr Ser Lys Asn Gln Leu
   65 70 75 80
   Ser Leu Gln Leu Arg Ser Val Thr Ala Ala Asp Thr Ala Val Tyr Tyr
   85 90 95
   Cys Ala Arg Ser Gly Tyr Asn Phe Trp Ser Gly Glu Tyr Tyr Gly Leu
   100 105 110
   Asp Ser Trp Gly Gln Gly Ala Val Val Thr Val Ser Ser
   115 120 125
<210> 10
   <211> 107
   <212> PRT
   <213> artificial
<220>
   <223> VL 6.20 variant
<400> 10
   Ala Ile Gln Leu Thr Gln Ser Pro Ser Ser Leu Ser Ala Tyr Val Gly
   1 5 10 15
   Asp Lys Val Thr Ile Thr Cys His Ala Ser Gln Gly Ile Asn Ser Trp
   20 25 30
   Leu Ala Trp Tyr Gln Gln Lys Pro Gly Lys Ala Pro Lys Leu Leu Ile
   35 40 45
   Tyr Lys Ala Ser Ser Leu Leu Ser Gly Val Pro Ser Arg Phe Ser Gly
   50 55 60
   Ser Gly Ser Gly Thr Asp Tyr Thr Leu Thr Ile Ser Ser Leu Gln Ser
   65 70 75 80
   Glu Asp Phe Ala Ser Tyr Tyr Cys Leu Gln Tyr Asp Ser Ala Pro Leu
                   85                  90                  95
   Ala Phe Gly Pro Gly Thr Lys Leu Asp Ile Lys
               100                 105
<210> 11
   <211> 125
   <212> PRT
   <213> artificial
<220>
   <223> VH J24.15 variant
<400> 11
   Gln Val Gln Leu Gln Glu Ser Gly Pro Gly Leu Leu Lys Pro Ser Glu
   1               5                   10                  15
   Thr Leu Ser Leu Thr Cys Ala Val Ser Gly Asp Ser Ile Ser Gly Gly
               20                  25                  30
   Tyr Tyr Trp Ser Trp Ile Arg Gln Ser Pro Gly Lys Gly Leu Glu Trp
           35                  40                  45
   Ile Gly His Ile Tyr Gly Ser Thr Ala Asp Thr Arg Tyr Asn Pro Ser
       50                  55                  60
   Leu Lys Ser Arg Val Thr Ile Ser Lys Asp Thr Ser Lys Asn Gln Leu
   65                  70                  75                  80
   Ser Leu Gln Leu Arg Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Leu Trp Gly Gln Gly Ala Val Val Thr Val Ser Ser
           115                 120                 125
<210> 12
   <211> 107
   <212> PRT
   <213> artificial
<220>
   <223> VL J24.15 variant
<400> 12
   Ala Ile Gln Leu Thr Gln Ser Pro Ser Ser Leu Ser Ala Tyr Val Gly
   1               5                   10                  15
   Asp Lys Val Thr Ile Thr Cys His Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Arg Ser Leu Gln Ser Gly Val Pro Ser Arg Phe Ser Gly
       50                  55                  60
   Ser Gly Ser Gly Thr Asp Tyr Thr Leu Thr Ile Ser Ser Leu Gln Ser
   65                  70                  75                  80
   Glu Asp Phe Ala Ser Tyr Tyr Cys Leu Gln Tyr Asp Ser Ala Pro Leu
                   85                  90                  95
   Ala Phe Gly Pro Gly Thr Lys Leu Asp Ile Lys
               100                 105
<210> 13
   <211> 125
   <212> PRT
   <213> artificial
<220>
   <223> VH J24.7 variant
<400> 13
   Gln Val Gln Leu Gln Glu Ser Gly Pro Gly Leu Leu Lys Pro Ser Glu
   1               5                   10                  15
   Thr Leu Ser Leu Thr Cys Ala Val Ser Gly Asp Ser Ile Ser Gly Gly
               20                  25                  30
   Tyr Tyr Trp Ser Trp Ile Arg Gln Ser Pro Gly Lys Gly Leu Glu Trp
           35                  40                  45
   Ile Gly His Ile Tyr Gly Ser Thr Ala Asp Thr Arg Tyr Asn Pro Ser
       50                  55                  60
   Leu Lys Ser Arg Val Thr Ile Ser Lys Asp Thr Ser Lys Asn Gln Leu
   65                  70                  75                  80
   Ser Leu Gln Leu Arg Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
                   100                 105                 110
   Asp Ser Trp Gly Gln Gly Ala Val Val Thr Val Ser Ser
           115                 120                 125
<210> 14
   <211> 107
   <212> PRT
   <213> artificial
<220>
   <223> VL J24.15 variant
<400> 14
   Ala Ile Gln Leu Thr Gln Ser Pro Ser Ser Leu Ser Ala Tyr Val Gly
   1               5                   10                  15
   Asp Lys Val Thr Ile Thr Cys Arg Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Gln Glu Gly Val Pro Ser Arg Phe Ser Gly
       50                  55                  60
   Ser Gly Ser Gly Thr Asp Tyr Thr Leu Thr Ile Ser Ser Leu Gln Ser
   65                  70                  75                  80
   Glu Asp Phe Ala Ser Tyr Tyr Cys Leu Gln Tyr Asp Ser Ala Pro Leu
                   85                  90                  95
   Ala Phe Gly Pro Gly Thr Lys Leu Asp Ile Lys
               100                 105
<210> 15
   <211> 125
   <212> PRT
   <213> artificial
<220>
   <223> VH V2 variant humanisé
<400> 15
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Asp Ser Ile Ser Ser Gly
               20                  25                  30
   Tyr Tyr Trp Ser Trp Ile Arg Gln Pro Pro Gly Lys Gly Leu Glu Trp
           35                  40                  45
   Ile Gly His Ile Tyr Gly Ser Thr Ala Asp Thr Asn Tyr Asn Pro Ser
       50                  55                  60
   Leu Arg Ser Arg Val Thr Ile Ser Lys Asp Thr Ser Lys Asn Gln Phe
   65                  70                  75                  80
   Ser Leu Gln Leu Ser Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Ser Trp Gly Gln Gly Thr Leu Val Thr Val Ser Ser
           115                 120                 125
<210> 16
   <211> 107
   <212> PRT
   <213> artificial
<220>
   <223> VL V2 variant humanisé
<400> 16
   Ala Ile Gln Leu Thr Gln Ser Pro Ser Ser Leu Ser Ala Ser Val Gly
   1               5                   10                  15
   Asp Arg Val Thr Ile Thr Cys Arg Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Tyr Asp Ser Ala Pro Leu
                   85                  90                  95
   Ala Phe Gly Pro Gly Thr Lys Val Asp Ile Lys
                   100                 105
<210> 17
   <211> 125
   <212> PRT
   <213> artificial
<220>
   <223> VH 6.20 variant humanisé
<400> 17
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Asp Ser Ile Ser Gly Gly
               20                  25                  30
   Tyr Tyr Trp Ser Trp Ile Arg Gln Pro Pro Gly Lys Gly Leu Glu Trp
           35                  40                  45
   Ile Gly Leu Ile Tyr Gly Ser Thr Ala Asp Thr Asn Tyr Asn Pro Ser
       50                  55                  60
   Leu Lys Gly Arg Val Thr Ile Ser Lys Asp Thr Ser Lys Asn Gln Phe
   65                  70                  75                  80
   Ser Leu Gln Leu Ser Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Ser Trp Gly Gln Gly Thr Leu Val Thr Val Ser Ser
           115                 120                 125
<210> 18
   <211> 107
   <212> PRT
   <213> artificial
<220>
   <223> VL 6.20 variant humanisé
<400> 18
   Ala Ile Gln Leu Thr Gln Ser Pro Ser Ser Leu Ser Ala Ser Val Gly
   1               5                   10                  15
   Asp Arg Val Thr Ile Thr Cys Arg Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Leu Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Tyr Asp Ser Ala Pro Leu
                   85                  90                  95
   Ala Phe Gly Pro Gly Thr Lys Val Asp Ile Lys
               100                 105
<210> 19
   <211> 125
   <212> PRT
   <213> artificial
<220>
   <223> VH J24.15 variant humanisé
<400> 19
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Asp Ser Ile Ser Gly Gly
               20                  25                  30
   Tyr Tyr Trp Ser Trp Ile Arg Gln Pro Pro Gly Lys Gly Leu Glu Trp
           35                  40                  45
   Ile Gly His Ile Tyr Gly Ser Thr Ala Asp Thr Asn Tyr Asn Pro Ser
       50                  55                  60
   Leu Lys Ser Arg Val Thr Ile Ser Lys Asp Thr Ser Lys Asn Gln Phe
   65                  70                  75                  80
   Ser Leu Gln Leu Ser Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Leu Trp Gly Gln Gly Thr Leu Val Thr Val Ser Ser
             115                 120                 125
<210> 20
   <211> 107
   <212> PRT
   <213> artificial
<220>
   <223> VL J24.15 variant humanisé
<400> 20
   Ala Ile Gln Leu Thr Gln Ser Pro Ser Ser Leu Ser Ala Ser Val Gly
   1               5                   10                  15
   Asp Arg Val Thr Ile Thr Cys Arg Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Arg Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Tyr Asp Ser Ala Pro Leu
                   85                  90                  95
   Ala Phe Gly Pro Gly Thr Lys Val Asp Ile Lys
               100                 105
<210> 21
   <211> 125
   <212> PRT
   <213> artificial
<220>
   <223> VH J24.7 variant humanisé
<400> 21
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Asp Ser Ile Ser Gly Gly
               20                  25                  30
   Tyr Tyr Trp Ser Trp Ile Arg Gln Pro Pro Gly Lys Gly Leu Glu Trp
           35                  40                  45
   Ile Gly His Ile Tyr Gly Ser Thr Ala Asp Thr Asn Tyr Asn Pro Ser
       50                  55                  60
   Leu Lys Ser Arg Val Thr Ile Ser Lys Asp Thr Ser Lys Asn Gln Phe
   65                  70                  75                  80
   Ser Leu Gln Leu Ser Ser Val Thr Ala Ala Asp Thr Ala Val Tyr Tyr
                   85                  90                  95
   Cys Ala Arg Ser Gly Tyr Asn Phe Trp Ser Gly Glu Tyr Tyr Gly Leu
               100                 105                 110
   Asp Ser Trp Gly Gln Gly Thr Leu Val Thr Val Ser Ser
           115                 120                 125
<210> 22
   <211> 107
   <212> PRT
   <213> artificial
<220>
   <223> VL J24.7 variant humanisé
<400> 22
   Ala Ile Gln Leu Thr Gln Ser Pro Ser Ser Leu Ser Ala Ser Val Gly
   1               5                   10                  15
   Asp Arg Val Thr Ile Thr Cys Arg Ala Ser Gln Gly Ile Asn Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Gln Glu Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Tyr Asp Ser Ala Pro Leu
                   85                  90                  95
   Ala Phe Gly Pro Gly Thr Lys Val Asp Ile Lys
               100                 105